# EUROPEAN PATENT SPECIFICATION

(11) **EP 1 322 316 B1**
(45) Date of publication and mention of the grant of the patent: **01.07.2009**
(21) Application number: 01967534.7
(22) Date of filing: 21.09.2001
(51) Int. Cl.: A61K 31/635, A61K 31/64, A61K 31/549, A61P 27/02, A61P 31/20, A61P 31/22

(54) **DIURETIC OR SULPHONYLUREA FOR USE IN ANTIVIRAL TREATMENT**
DIURETIKUM ODER SULFONYLHARNSTOFF ZUR BENUTZUNG IN ANTIVIRALER BEHANDLUNG
DIURETIQUE OU SULPHONYLUREE UTILISE DANS LE TRAITEMENT ANTIVIRAL

(30) Priority: 21.09.2000 GB 0023199; 19.02.2001 GB 0104030; 19.02.2001 GB 0104031; 19.06.2001 GB 0114947
(43) Date of publication of application: 02.07.2003
(73) Proprietor: Henderson Morley Research and Development Limited, Birmingham B13 8JS (GB)
(72) Inventor: PARDOE, Ian Stuart, Henderson Morley Res.& Dev.Ltd, Birmingham B13 8JS (GB); HARTLEY, Christopher Edward, Halesowen B63 4QR (GB)
(74) Representative: Moore, Christopher Mark
(86) International application number: PCT/GB2001/004206
(87) International publication number: WO 2002/024207

(56) References cited:
- WO-A-01/49242
- WO-A-01/49300
- REY ET AL: "effets inhibiteurs de l'hydrochlorothiazide sur la primo-infection herpétique de l'enfant africain" BULLETIN DE L'ACADÉMIE NATIONALE DE MEDECINE, vol. 148, 1964, pages 360-364, XP001055317
- FELTEN G.: "Erfahrungen mit Furosemid in der Behandlung des Zoster ophthalmicus" HAUTARZT, vol. 23, no. 3, 1972, pages 140-142, XP001056345
- DATABASE WPI Week 37, 1995 Derwent Publications Ltd., London, GB; AN 280839 XP002190114 SHIRAKI ET AL: "antiviral agent effective against resistant herpes virus containing 9-2-hydroxy-ehtoxy-methyl-guanine and Geum japonica Thunb. extract" & JP 07 179347 A (SHOWA SHELL SEKIYU KK)
- DATABASE WPI Week 49, 2000 Derwent Publications Ltd., London, GB; AN 533315 XP002190115 DE LACERDA MARQUES SOUZA: "herpes treatment composition consists of a mixture of an extract or dilute tincture or active base" & BR 9 804 577 A (DE LACERDA MARQUES SOUZA), 8 August 2000 (2000-08-08)
- VOSS T G ET AL: "REDUCTION OF HUMAN IMMUNODEFICIENCY VIRUS PRODUCTION AND CYTOPATHICEFFECTS BY INHIBITORS OF THE NA+/K+/2CI- COTRANSPORTER" VIROLOGY, ACADEMIC PRESS,ORLANDO, US, vol. 219, no. 249, 1996, pages 291-294, XP002925373 ISSN: 0042-6822

## Description

The invention relates to anti-viral treatments and in particular to prophylactic and therapeutic treatments of DNA viral infections such as Herpes virus infections.

Herpes viruses are DNA viruses, having a central core of DNA within a proteinaceous structure DNA carries the genetic code to reproduce the virus. Viruses must infect a living cell to reproduce. There are numerous viral proteins that are well characterised including important enzymes which act as ideal targets for antiviral chemotherapy. These include DNA polymerase and thymidine kinase which are needed for DNA replication. The replication of viral DNA is essential for virus infectivity. It is known that infecting viruses can alter the natural ionic balances of a living cell in the course of their replication.

Rey et a/ (Bulletin de l'académie de médicine, 1964, 148) have discovered that hydrochlorothiazide administered orally had an indirect virostatic effect.

Felton (Hantarzt, 1972, 23(3)) discloses that administering furosemide orally can treat oedema associated with zoster ophthalmicus.

We have discovered that certain classes of known drugs can be used for an antiviral effect against DNA viruses.

According to this invention in one aspect there is provided the use of a diuretic for the manufacture of a topical medicament for the treatment of DNA viral infections wherein the diuretic is selected from one of:
- a loop diuretic;
- a thiazide diuretic; or
- a sulphonylurea
and wherein the diuretic is present in an effective anti-viral amount at a level to alter the natural ionic balance of a cell to a level less than that which will affect cellular metabolism detrimentally but sufficient to inhibit replication of viral DNA and the diuretic is the only agent effective against DNA viruses.

Loop diuretics are substances which act on the ascending loop of Henlé in the kidney. They are sulphonamides but may be other substances too. Typical examples include:

| | |
|---|---|
| acetazolamide | mefruside |
| ambuside | methazolamide |
| azosemide | piretanide |
| bumetanide | torsemide |
| butazolamide | tripamide |
| chloraminophenamide | xipamide |
| clofenamide | |
| clopamide | ethacrynic acid |
| clorexolone | etozolin |
| disulfamide | ticrynafen |
| ethoxzolamide | |
| furosemide | |

Preferably the loop diuretic is one or more of frusemide, bumetamide, ethacymic acid or torasemide.

Preferred is frusemide which is an anthrilic acid derivative, chemically 4-chloro-N-furfuryl-5-sulfamoylanthranilic acid. It is practically insoluble in water at neutral pH, however is freely soluble in alkali. Frusemide exerts its physiological effect by inhibition of the transport of chloride ions across cell members. Frusemide is a loop diuretic with a short duration of action. It is used for treating oedema due to hepatic, renal, or cardiac failure and treating hypertension. The bioavailability of frusemide is between 60% to 70% and it is primarily excreted by filtration and secretion as unchanged drug. Frusemide acts on the Na+/K+/2CI- cotransformer. For its diuretic effect, its predominant action is in the ascending limb of the loop of Henlé in the kidney. Loop diuretics markedly promote K⁺ excretion, leaving cells depleted in intracellular potassium. This may lead to the most significant complication of long term systemic frusemide usage namely a lowered serum potassium. We postulate that it is this action however which makes frusemide a candidate for use as an agent against DNA viral infections.

Recent evidence suggests that the major biotransformation product of frusemide is a glucuronide. Frusemide is extensively bound to plasma proteins, mainly albumin. Plasma concentrations ranging from 1 to 400 mcg/ml are 91-99% bound in healthy individuals. The unbound fraction ranges between 2.3-4.1% at therapeutic concentrations. The terminal half life of frusemide is approximately 2 hours, and it is predominantly excreted in the urine.

Thiazide diuretics include the benzothiadriazines derivatives, also known as thiazides. Typical examples are:

| | |
|---|---|
| althiazide | hydrobenzthiazide |
| bemetizide | hydrochlorothiazide |
| bendroflumethiazide | hydrofluoromethiazide |
| benzthiazide | indapamide |
| benzylhydrochlorothiazide | mebutizide |
| buthiazide | methylcyclothiazide |
| chlorothiazide | meticane |
| chlorothalidone | metalazone |
| cyclopenthiazide | paraflutizide |
| cyclothiazide | polythiazide |
| epithiazide | quinethazone |
| ethiazide | teclothiazide |
| fenquizone | trichlormethiazide |

Preferably the thiazide diuretic is one or more of chlorothiazide, hydrochlorothiazide, hydroflumethiazide, methyclothiazide, trichlormethazide, benzthiazide, bendroflumethazide, bendrofluazide, polythiazide or cyclothiazide.

Sulphonylureas are anti-diabetic drugs which influence ion transport across cell membranes. They are instanced by:

| | |
|---|---|
| acetohexamide | glyburide |
| 1-butyl-3-metanilylurea | glybuthiazole |
| carbutamide | glybuzole |
| chlorpropamide | glycycloamide |
| glibenclamide | glyclopyramide |
| glibomuride | glyhexamide |
| gliclazide | glymidine |
| glimepiride | glypinamide |
| glipizide | phenbutamide |
| gliquidone | tolazamide |
| glisentide | tolbutamide |
| glisolamide | tolcylamide |
| glisoxepid | |

Preferably the sulphonylurea is one or more of tolbutamide, tolazamide, tolcyclamide, glibornuridum, acetohexamide, chlorpropamide, carbutamide, glyburide or glipizide

By altering the cellular concentrations of ions, cellular ionic balances, cellular ionic milieu and cellular electrical potentials by the application of a diuretic or a sulphonylurea it is possible to change the metabolism of the cell without detriment to the cell but so that virus replication is inhibited. Anti-viral efficacy has been demonstrated against the DNA viruses Herpes simplex virus type 1 and type 2, Feline Herpes virus, Cyclomegalo virus, Varicella zoster virus and Pseudorabies and Adenoviruses.

In another aspect the invention provides an anti DNA viral infection topical composition for the treatment of DNA viral infections in subjects, the topical composition comprising an effective anti-viral amount of a diuretic in a carrier therefore, the diuretic being selected from one of:
- a thiazide diuretic; or
- a sulphonylurea
wherein the diuretic is the only agent effective against DNA viruses present.

The compositions of the invention may be adapted for slow release. Other ingredients may be present, provided that they do not compromise the anti-viral activity.

A preferred concentration of loop diuretic is 300 µg in a liquid carrier.

A preferred concentration of thiazide diuretic is from 0.01 mg/ml to 5.0 mg/ml in a liquid carrier.

A preferred concentration of sulphonylurea is from 0.5 mg/ml and 5 mg/ml in a liquid carrier.

A preferred embodiment of this invention is the use of local concentrations of a loop diuretic or sulphonylurea as a highly effective treatment of virus infections of the eye. Recurrent Herpes infections of the cornea in man is the most common viral cause of blindness.

In order that the invention may be well understood it will now be described by way of illustration only with reference to the following examples:

### EXAMPLE I

In vitro bioassays were undertaken to follow the anti-viral activity of a diuretic compound.

The compositions of frusemide and a carrier were applied to African green monkey kidney and BHK1 veros cells infected with type 2 herpes simplex virus (strains 3345 and 180) at low, intermediate, and high multiplicities of infection (MOI). Inhibition of virus replication was scored on the scale:

| | |
|---|---|
| no inhibition | - |
| 20% inhibition | + |
| 40% inhibition | ++ |
| 60% inhibition | +++ |
| 80% inhibition | ++++ |
| 100% inhibition | +++++ |
| T denotes drug toxicity. | |

The following results were obtained using African green monkey kidney cells and type 2 herpes simplex strain 3345:

| Inhibition of hsv2 | |
|---|---|
| **Multiplicity of infection (Dose of virus)** | **Effect of frusemide** |
| High | - |
| Medium | ++ |
| Low | ++ |

The experiment was repeated using BHK1 vero cells and type 2 herpes simplex strain 180. Similar results were obtained.

These results show the antiviral effect of frusemide at 1 mg/ml.

### EXAMPLE II

In vitro bioassays were undertaken to determine the anti-viral activity of a thiazide diuretic compound, in the method of Example I.

| Inhibition of hsv2 | |
|---|---|
| **Multiplicity of Infection (Dose of virus)** | **Effect of bendrofluazide (0.25mg bendrofluazide** / **ml liquid Medium)** |
| High | ++ |
| Medium | ++++ |
| Low | ++++ |

The experiment was repeated using BHK1 vero cells and type 2 herpes simplex strain 186. Similar results were obtained.

### EXAMPLE III

In vitro bioassays were undertaken to follow the anti-viral activity of a sulphonylurea compound, in the method of Example 1.

The following results show the effect of a range of concentrations of tolbutamide over a range of multiplicities of infection using hsv2 :

| | **50mg tolbutamide / ml liquid medium** | **5mg tolbutamide, / ml liquid medium** | **0.5mg tolbutamide/ml liquid medium** | **0mg tolbutamide / ml tolbutamide** |
|---|---|---|---|---|
| **High multiplicity of infection** | +++ | ++ | + | - |
| **Medium multiplicity of infection** | ++++ | +++ | + | - |
| **Low multiplicity of infection** | +++++ | +++ | ++ | - |

The experiment was repeated using BHK1 vero cells and type 2 herpes simplex strain 186. Similar results were obtained.

## Claims

1. Use of a diuretic for the manufacture of a topical medicament for the treatment of DNA viral infections
wherein the diuretic is selected from one of:
• a loop diuretic;
• a thiazide diuretic; or
• a sulphonylurea
and wherein the diuretic is present in an effective anti-viral amount at a level to alter the natural balance of a cell to a level less than that which will affect cellular metabolism detrimentally but sufficient to inhibit replication of viral DNA and the diuretic is the only agent effective against DNA viruses.

2. Use according to Claim 1, wherein the diuretic is a loop diuretic.

3. Use according to Claim 2, wherein the loop diuretic is one or more of frusemide, bumetamide, ethacyrnic acid or torasemide.

4. Use according to Claim 3, wherein the loop diuretic is frusemide.

5. Use according to Claim 1, wherein the diuretic is a thiazide diuretic.

6. Use according to Claim 5, wherein the thiazide diuretic is one or more of chlorothiazide, hydrochlorothiazide, hydroflumethiazide, methyclothiazide, trichlormethazide, benzthiazide, bendroflumethazide, bendrofluazide, polythiazide or cyclothiazide.

7. Use according to Claim 1, wherein the sulphonylurea is one or more of tolbutamide, tolazamide, tolcyclamide, glibornuridum, acetohexamide, chlorpropamide, carbutamide, glyburide or glipizide.

8. An anti DNA viral infection topical composition for the treatment of DNA viral infections in subjects, the topical composition comprising an effective anti-viral amount of a diuretic in a carrier therefor, the diuretic being selected from one of:
• a thiazide diuretic; or
• a sulphonylurea
wherein the diuretic is the only agent effective against DNA viruses present.

9. A composition according to Claim 8, wherein the diuretic is a thiazide diuretic.

10. A composition according to Claim 9 wherein the thiazide diuretic is one or more of chlorothiazide, hydrochlorothiazide, hydroflumethiazide, methyclothiazide, trichlormethazide, benzthiazide, bendroflumethazide, bendrofluazide, polythiazide or cyclothiazide.

11. A composition according to Claim 8, wherein the diuretic is a sulphonylurea selected from tolbutamide, tolazamide, tolcyclamide, glibornuridum, acetohexamide, chlorpropramide, carbutamide, glybumide or glypizide.

## Patentansprüche

1. Verwendung eines Diuretikums zur Herstellung eines topischen Medikaments zur Behandlung von DNA-Virusinfektionen,
wobei das Diuretikum ausgewählt ist aus einem von:
• einem Schleifendiuretikum;
• einem Thiazid-Diuretikum; oder
• einem Sulfonylharnstoff
und wobei das Diuretikum in einer wirksamen antiviralen Menge bei einem solchen Spiegel vorliegt, dass das natürliche Gleichgewicht einer Zelle zu einem Spiegel verändert wird, der niedriger ist als der, welcher den Zellmetabolismus nachteilig beeinflussen wird, aber ausreichend ist, um die Replikation von viraler DNA zu inhibieren, und das Diuretikum das einzige Mittel ist, das gegen DNA-Viren wirksam ist.

2. Verwendung gemäß Anspruch 1, wobei das Diuretikum ein Schleifendiuretikum ist.

3. Verwendung gemäß Anspruch 2, wobei das Schleifendiuretikum eines oder mehrere von Frusemid, Bumetamid, Ethacrynsäure oder Torasemid ist.

4. Verwendung gemäß Anspruch 3, wobei das Schleifendiuretikum Frusemid ist.

5. Verwendung gemäß Anspruch 1, wobei das Diuretikum ein Thiazid-Diuretikum ist.

6. Verwendung gemäß Anspruch 5, wobei das Thiazid-Diuretikum eines oder mehrere von Chlorthiazid, Hydrochlorthiazid, Hydroflumethiazid, Methyclothiazid, Trichlormethazid, Benzthiazid, Bendroflumethazid, Bendrofluazid, Polythiazid oder Cyclothiazid ist.

7. Verwendung gemäß Anspruch 1, wobei der Sulfonylharnstoff eines oder mehrere von Tolbutamid, Tolazamid, Tolcyclamid, Glibornuridum, Acetohexamid, Chlorpropamid, Carbutamid, Glyburid oder Glipizid ist.

8. Eine topische Zusammensetzung gegen DNA-Virusinfektionen zur Behandlung von DNA-Virusinfektionen bei Subjekten, wobei die topische Zusammensetzung eine wirksame antivirale Menge eines Diuretikums in einem Träger für dieses umfasst, wobei das Diuretikum ausgewählt ist aus einem von:
• einem Thiazid-Diuretikum; oder
• einem Sulfonylharnstoff
wobei das Diuretikum das einzige Mittel ist, das gegen vorhandene DNA-Viren wirksam ist.

9. Eine Zusammensetzung gemäß Anspruch 8, wobei das Diuretikum ein Thiazid-Diuretikum ist.

10. Eine Zusammensetzung gemäß Anspruch 9, wobei das Thiazid-Diuretikum eines oder mehrere von Chlorthiazid, Hydrochlorthiazid, Hydroflumethiazid, Methyclothiazid, Trichlormethazid, Benzthiazid, Bendroflumethazid, Bendrofluazid, Polythiazid oder Cyclothiazid ist.

11. Eine Zusammensetzung gemäß Anspruch 8, wobei das Diuretikum ein Sulfonylharnstoff ist, der ausgewählt ist aus Tolbutamid, Tolazamid, Tolcyclamid, Glibornuridum, Acetohexamid, Chlorpropamid, Carbutamid, Glyburid oder Glypizid.

## Revendications

1. Utilisation d'un diurétique pour la fabrication d'un médicament topique pour le traitement d'infections virales à ADN
dans laquelle le diurétique est choisi parmi l'un des éléments suivants :
• un diurétique de l'anse de Henle ;
• un diurétique thiazide ; ou
• une sulfonylurée
et dans laquelle le diurétique est présent en une quantité antivirale efficace à un niveau tel à modifier l'équilibre naturel d'une cellule jusqu'à un niveau inférieur à celui qui affecterait le métabolisme cellulaire de façon préjudiciable, mais suffisant pour inhiber la réplication de l'ADN viral et le diurétique est le seul agent efficace contre les virus à ADN.

2. Utilisation selon la revendication 1, dans laquelle le diurétique est un diurétique de l'anse de Henle.

3. Utilisation selon la revendication 2, dans laquelle le diurétique de l'anse de Henle est un ou plusieurs éléments parmi le frusémide, le bumétamide, l'acide éthacymique ou le torasémide.

4. Utilisation selon la revendication 3, dans laquelle le diurétique de l'anse de Henle est le frusémide.

5. Utilisation selon la revendication 1, dans laquelle le diurétique est un diurétique thiazide.

6. Utilisation selon la revendication 5, dans laquelle le diurétique thiazide est ou un ou plusieurs éléments parmi le chlorothiazide, l'hydrochlorothiazide, l'hydrofluméthiazide, le méthyclothiazide, le trichlorméthazide, le benzthiazide, le bendrofluméthazide, le bendrofluazide, le polythiazide ou le cyclothiazide.

7. Utilisation selon la revendication 1, dans laquelle la sulfonylurée est un ou plusieurs éléments parmi le tolbutamide, le tolazamide, le tolcyclamide, le glibomuridum, l'acétohexamide, le chlorpropamide, le carbutamide, le glyburide ou le glipizide.

8. Composition topique pour infection virale anti-ADN destinée au traitement d'infections virales à ADN chez des sujets, la composition topique comprenant une quantité antivirale efficace d'un diurétique dans un support destiné à cet effet, le diurétique étant choisi parmi l'un des éléments suivants :
• un diurétique thiazide ; ou
• une sulfonylurée
dans laquelle le diurétique est le seul agent efficace contre les virus à ADN présents.

9. Composition selon la revendication 8, dans laquelle le diurétique est un diurétique thiazide.

10. Composition selon la revendication 9, dans laquelle le diurétique thiazide est ou un ou plusieurs éléments parmi le chlorothiazide, l'hydrochlorothiazide, l'hydrofluméthiazide, le méthyclothiazide, le trichlorméthazide, le benzthiazide, le bendrofluméthiazide, le bendrofluazide, le polythiazide ou le cyclothiazide.

11. Composition selon la revendication 8, dans laquelle le diurétique est une sulfonylurée choisie parmi le tolbutamide, le tolazamide, le tolcyclamide, le glibomuridum, l'acétohexamide, le chlorpropamide, le carbutamide, le glybumide ou le glipizide.
